(19) Europäisches Patentamt
European Patent Office
Office européen des brevets

(11) Publication number: 0 123 650 B1

(12) EUROPEAN PATENT SPECIFICATION

(45) Date of publication of patent specification: 28.10.87

(51) Int. Cl.$^4$: C 07 D 499/00, A 61 K 31/43

(21) Application number: 84810146.5

(22) Date of filing: 26.03.84

(54) **Penems, pharmaceutical compositions containing them, and process for their preparation.**

(30) Priority: 25.03.83 US 478620

(43) Date of publication of application:
31.10.84 Bulletin 84/44

(45) Publication of the grant of the patent:
28.10.87 Bulletin 87/44

(84) Designated Contracting States:
AT BE CH DE FR GB IT LI LU NL SE

(56) References cited:
EP-A-0 002 210
EP-A-0 070 204

(73) Proprietor: SCHERING CORPORATION
2000 Galloping Hill Road
Kenilworth, New Jersey 07033 (US)

(72) Inventor: Giryavallabhan, Vyyoor Moopil
10 Maplewood Drive
Parsippany New Jersey 07054 (US)
Inventor: Ganguly, Ashit Kumar
96 Cooper Avenue
Upper Montclair New Jersey 07043 (US)
Inventor: Pinto, Patrick Anthony
232 Randolph Avenue
Mine Hill New Jersey 07801 (US)
Inventor: Versace, Richard William
13 Edgewood Road
Ringwood New Jersey 07456 (US)

(74) Representative: Ritter, Stephen David et al
Mathys & Squire 10 Fleet Street
London EC4Y 1AY (GB)

**The file contains technical information submitted after the application was filed and not included in this specification**

EP 0 123 650 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a novel penems.

EP—A—70204 discloses a class of penems including examples substituted in the 6-position by hydroxyethyl and in the 2-position by substituents including substituents containing heterocyclic groups.

According to the present invention there are provided novel 6-(hydroxyalkyl)-2-heterocycloaliphatic-alkylthio)penems having the general formula:

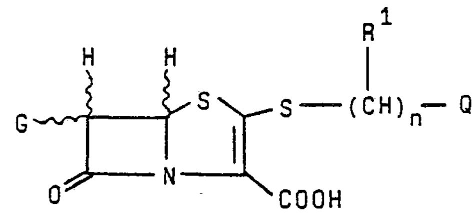

I

wherein

G is hydroxyloweralkyl,

Q is the group

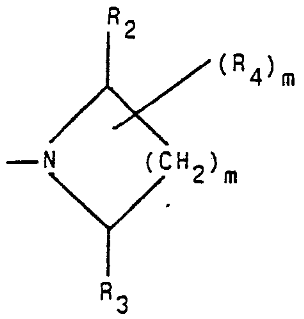

or

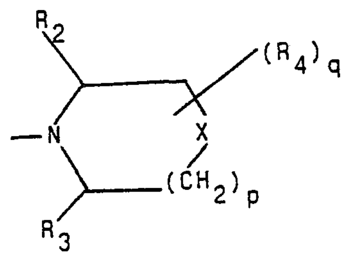

A    B

wherein:

each $R^1$ is independently lower alkyl, hydrogen, carboxy, carbamyl, cyano, hydroxy, amino, amino lower-alkylthio, fluoro, loweralkoxy, loweralkanoyloxy, or loweralkyl substituted by imidazolyl (which may be substituted by a group R defined below) amino, mono- or di-loweralkylamino, ureido, semicarbazido, hydroxy, cyano, halogen, loweralkoxy, carbamyloxy, carboxy, carbamyl, loweralkylcarbonyl, sulfo, sulfamyl, lower alkylsulfonyl, loweralkoxysulfonyl, loweralkoxycarbonyl hydroxyloweralkylcarbonyl or hydroxyloweralkylsulfonyl, with the proviso that when $R_1$ is attached to a carbon atom adjacent either to the sulphur atom connected to the 2-position of the penem ring or the nitrogen atom by which the group Q is connected to the side chain

$$-(\overset{\overset{\displaystyle R^1}{|}}{C}H)_n-$$

then such $R_1$ cannot be hydroxy, amino or fluoro;

R is hydrogen, loweralkyl, aminoloweralkyl, mono- and di-loweralkylaminoloweralkyl, carboxyloweralkyl, sulfoalkyl, hydroxyalkyl, cyano, hydroxy, amino, mono- and di-lowerlalkylamino, alkylsulfonate, sulfamyl, halogeno, hydroxyliminoloweralkyl, loweralkoxyimino-loweralkyl, carboxy, carbamyl, mono- or di-loweralkylcarbamyl, nitro, carbamyloxy, ureido, ureidoloweralkyl, or carbamylhydrazolower-alkyl;

$R_2$ and $R_3$ are independently selected from hydrogen, loweralkyl, aminoloweralkyl, mono- and di-loweralkylaminoloweralkyl, carboxyloweralkyl, carboxy, hydroxyloweralkyl, cyano, oxo, carbamyl, and mono- and di-loweralkylcarbamyl;

$R_4$ is chosen from the groups listed for $R_2$ and $R_3$, sulfoloweralkyl, hydroxy, amino, mono- and di-loweralkylamino, loweralkylsulfonate, sulfamyl, halogeno, hydroxyliminoloweralkyl and loweralkoxy-iminoloweralkyl, hydroxylimino, loweralkoxyimino

X is O, S, $SO_2$, $NR_2$ e.g. NH, or $NCOR_2$, wherein $R_2$ is as defined above,

m and n are 1 to 4

P is 1 or 2

q is 1, 2 or 3

and the pharmaceutically acceptable salts and esters thereof, in racemic or optically active form.

A preferred $R^1$ substituent is substituted loweralkyl e.g. hydroxymethyl or aminomethyl.

Preferably $R_2$ and $R_3$ are chosen from hydrogen and oxo.

Preferably $R_4$ is chosen from hydrogen, amino, hydroxy, oxo and carboxy.

Also preferred are compounds of formula I wherein n is 2 to 4; most preferred are those compounds wherein n is 2.

G, is preferably 1-hydroxyethyl.

A preferred compound is one in which Q is the grouping B, p is 1, $R_2$, $R_3$ and $R_4$ are hydrogen and X is

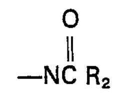

where $R_2$ is amino or loweralkylamino.

The term "oxo" refers to a doubly bonded oxygen atom which together with the carbon to which it is attached forms a carbonyl group.

The term "loweralkyl" as used herein means alkyl groups of 1 to 6 carbon atoms and includes methyl, ethyl, propyl, butyl, pentyl and hexyl and the corresponding branched chain isomers thereof. Similarly, "loweralkyl" means straight or branched alkoxy groups having 1 to 6 carbon atoms, e.g., methoxy, ethoxy, propoxy and butoxy, and "loweralkanoyloxy" means straight or branched chain alkanoyloxy groups of 1 to 6 carbon atoms, e.g. acetoxy, propionoxy, butyryloxy, isopropionoxy and isobutyryloxy.

Compounds of the present invention possess a number of asymmetric carbon atoms, indicated in the partial formula II below at the 5, 6, and possibly in the 2′ to 5′-position carbon atoms. Also where G is hydroxy lower-alkyl other than hydroxymethyl the carbon atom to which the hydroxy group is attached will be asymmetric.

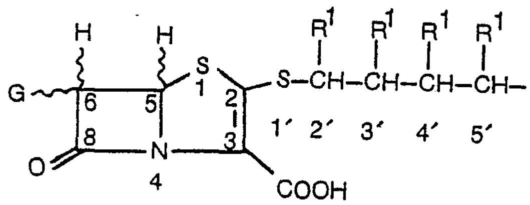

II

Where G is 1-hydroxyethyl the compounds of the invention preferably possess 5R, 6S, 8R or 5R, 6R, 8S stereochemistry at the indicated chiral atoms. The preferred absolute stereochemistry for such compounds of the present invention at those positions is 5R,6S,8R.

Compounds of formula I will have asymmetric carbon atoms(s) as shown in formula II at the 2′ to 5′ positions.

The present invention contemplates compounds of formula I in stereospecific form or as mixtures of such stereoisomers.

As used herein, "pharmaceutically acceptable salts" preferably means alkyli metal salts such as sodium and potassium salts; alkaline earth metal salts such as calcium, magnesium and aluminum salts; amine salts formed from a wide variety of suitable organic amines, i.e., aliphatic, cycloaliphatic, (cycloaliphatic)aliphatic or araliphatic primary, secondary or tertiary mono-, di or polyamines, or heterocyclic bases, e.g., salts derived from triethylamine, 2-hydroxyethylamine, di-(2-hydroxyethyl)amine, tri-(2-hydroxyethyl)amine, 4-aminobenzoic acid-2-diethylaminoethyl ester, 1-ethylpiperidine, bicyclohexylamine, N,N′-dibenzylethylenediamine, pyridine, collidine, quinoline, procaine, dibenzylamine, 1-ephenamine and N-alkylpiperidine; or acid addition salts formed from mineral acids such as hydrochloric, hydrobromic, hydroiodic, phosphoric or sulfuric acids, or formed from organic carboxylic or sulfonic acids such as trifluoroacetic, para-toluene sulfonic, maleic, acetic, citric, oxalic, succinic, benzoic, tartaric, fumaric, mamdelic, ascorbic and malic acids.

Compounds of this invention which contains a 3-carboxylic group and a basic group (which may be an $R^1$ group e.g. an amine), or the group Q (especially a group Q containing two nitrogen atoms) may form an inner salt i.e. a zwitterion.

"Pharmaceutically acceptable esters" means physiologically cleavable esters, i.e., metabolizable esters known in the penicillin, cephalosporin and penem arts to be easily cleaved within the body to the parent acid. Examples of groups forming such esters are indanyl, phthalidyl, methoxymethyl, glycyloxymethyl, phenylglycyloxymethyl, thienylglycyloxymethyl, acetoxymethyl and pivaloyloxymethyl.

When tested in standardized microbiological assay, the compounds of this invention are active against such gram-positive organisms as *Staphylococcus epidermis* and *Bacillus subtilis*, and such gram-negative organisms as *E. coli* and *Salmonella*, at test levels of 0.03 to 1.0 micrograms/ml. Additionally, they show activity against organisms which produce beta-lactamases, e.g. penicillinase and cephalosperinase, indicating a stability toward these enzymes *Staphylococcus* aureus 76010501 at a test level of 0.031

microgram/ml. When tested against *E. coli* 71120101 TEM—1 (a beta-lactamase producing organism) the compound exhibits activity at 0.500 microgram/ml.

The compounds of this invention and their metabolites have little or no unpleasant odour.

As antibacterial agents, the compounds of this invention are conventionally formulated for oral, parenteral, topical and transdermal use. Thus, this invention includes within its scope pharmaceutical compositions comprising the compounds of this invention in admixture with a pharmaceutically acceptable carrier therefor. In the foregoing compositions, the compounds of this invention can be used as the sole active antibacterial agent or in combination with other antibacterial agents and/or enzyme inhibitors.

For oral administration, the compounds of this invention are typically formulated in the form of tablets, capsules, elixirs, or the like. For parenteral administration they may be formulated into solutions or suspensions.

The dosage of a compound of this invention which is administered will be dependent on the judgment of the attending clinician taking into account a variety of factors, i.e., the age and weight of the individual being treated, the mode of administration, and the type and severity of the bacterial infection being prevented or reduced and the potency of the specific compound administered. Typically, the dosage administered per day will be in the range of from about 1 to 250 mg/kg and preferably from about 5 to 20 mg/kg in divided dosages.

Typically, the dosage will be administered in dosage units containing convenient amounts, for example, 125, 250 or 500 mg of active ingredient combined with a suitable physiologically acceptable carrier or diluent.

According to another aspect of the present invention a process for preparing a compound of formula I as defined above or its salts or esters, is characterized in that:

A) a compound of formula [IX(a), IX(b)]

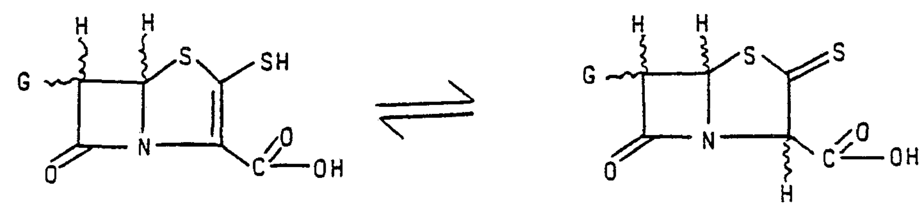

IX(a) IX(b)

in which G is as defined above, is transformed into a compound of formula I by introducing the group

$$-\underset{\displaystyle |}{\overset{R^1}{}}\!\!(CH)_n-Q$$

(in which Q, *n* and $R^1$ are as previously defined) by known conventional methods;

B) Reaction of a compound of the general formula XI

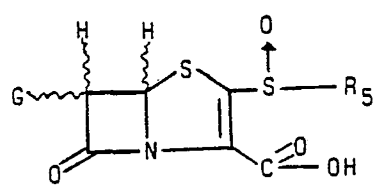

XI

in which G is as defiend above and R is an organic group, with a compound of general formula XII

$$HS-\overset{R^1}{\overset{|}{(CH)_n}}-Q \qquad XII$$

in which Q, *n* and R' are as previously defined, or with a reactive derivative thereof, wherein the group R is different from the group

$$-\overset{R^1}{\overset{|}{(CH)_n}}-Q$$

by which it is replaced,

C) intramolecular cyclisation of a compound having the general formula XIII

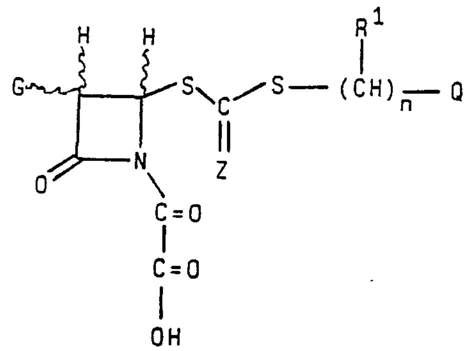

XIII

in which G, n, $R^1$ and Q are as defined above and Z is oxygen or sulphur, in the presence of a trivalent organophosphorous compound;

D) for the preparation of a compound of formula I in which at least one $R^1$ is cyano or fluoro, converting at least one hydroxy group representing $R^1$ in an compound of formula I into the cyano or fluoro group; wherein in the processes A, B, C or D, any functional groups are protected if necessary or desired, the process A, B, C, or D, being followed by removal of any protecting groups, before or after any desired separation of a stereoisomer, and isolation of the resulting penem compound of formula I as the free acid, pharmaceutically acceptable salt or pharmaceutically acceptable ester.

Process A):

The transformation of tautomer [IX(a), IX(b)] to a compound of formula I is preferably carried out using a process similar to one or other of the transformation processes described in EP—A—110 280, that is by:

(i) reaction with a compound of formula X

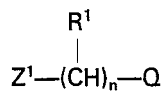

X

in which W, $R^1$ and n are as defined above and $Z^1$ is a group leaving under the conditions of the reaction, e.g. activated hydroxy, halogen, $CH_3SO_2—O—$, $O^-—P^+(C_6H_5)_3$, or the group $CF_3SO_3—$. Suitable halogens are chlorine, bromine and iodine.

Such reaction is preferably carried out in an organic solvent such as tetrahydrofuran in an inert atmosphere at a temperature typically beween −5°C and 30°C. The reaction will generally be carried out in the presence of a base or acid acceptor, e.g. an inorganic carbonate, and the reaction will be generally complete within 1 to 3 hours.

(ii) Reaction with a 1,2-epoxy compound of the formula X′

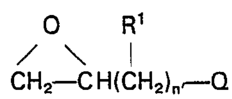

X′

in which Q and $R^1$ are as defined above and n′ is 0 to 2.

This reaction can be used for producing compounds of formula I in which $R^1$ group attached to the second carbon atom from the sulphur atom connected to the 2-position of the penem ring is hydroxy, i.e. compounds having the partial structure

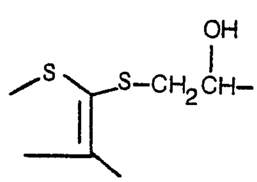

Such reaction can be carried out by combining the tautomer and the compound of formula X′ at, or about at room temperature, usually in a suitable inert solvent such as dimethylformamide.

Procedure B) involving sulfoxide replacement can be carried out, usually in an inert solvent, e.g. dichloromethane or THF. The reaction temperature is usually in the range of 0°C to −70°C.

When the thiol compound XII is itself used, the reaction is generally carried out in the presence of a base, e.g. or organic base e.g. diisopropylethylamine or triethylamine, or an inorganic base, e.g. potassium hydroxide or sodium methoxide.

Alternatively a reactive derivative, e.g. an alkali metal salt, preferably sodium or potassium may be used.

The sulfoxides of formula XI can be obtained by treating a compound of the formula XI'.

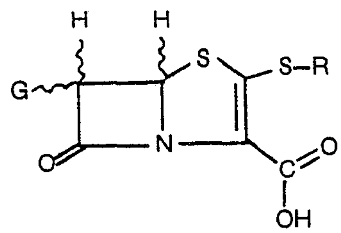

XI

in which G and R are as defined above, with a mild oxidizing agent e.g. peroxybenzoic acid in an inert solvent, e.g. dichloromethane, at between −30°C and 20°C e.g. 0°C to 5°C.

A compound of formula XI' can be prepared by the method disclosed in our European Patent Specification Publication No. 13662 or by the methods disclosed herein.

Procedure, C), involving cyclisation of the compound of formula XIII will generally be carried out analogously to the process described in our European Patent Application, Publication No. 58317.

Thus it is usually carried out in an inert solvent, for example an aromatic hydrocarbon, e.g. toluene, benzene, aliphatic ethers e.g. diethyl ethers and dipropyl ether, cyclic ethers e.g. dioxane and tetrahydrofuran and halogenated hydrocarbons e.g. methylene chloride and chloroform.

In general the cyclization reaction is conducted at temperatures in the range from 20°C to 80°C, usually from 40°C to 60°C for a period of from 6 to 24 hours.

Suitable trivalent organophosphorous compounds are cyclic and/or acyclic trialkylphosphites, triaryl phosphites and mixed arylalkylphosphites or phosphoramides. The preferred trivalent organophosphorous compound is a trialkylphosphite; most preferred is triethylphosphite.

Process D can be carried out by methods known in the art for replacing the hydroxy of hydroxyalkyl groups with fluoro or cyano.

A convenient general procedure is to react the hydroxy side chain with dialkylazido carboxylate (DAC) and triphenyl phosphine (TPP) to convert the hydroxy to a leaving group, which can then be displaced with fluoro or cyano anion. For example, a suitably protected penem in solution in a suitable inert solvent such as methylene chloride can be added to the DAC and TPP reagents under cooling e.g. −30°C to −10°C preferably −20°C. A fluoro or cyano ion source can then be added, for example sodium or potassium cyaninde, or diphenoxycyanophosphine, or a solution of hydrogen fluoride in pyridine.

Alternatively, for example, a suitably protected penem can be reacted with a dialkylaminosulfur trifluoride preferably diethylaminosulfur trifluoride, usually in the presence of an inorganic base, e.g. calcium carbonate, to convert the hydroxy to fluoro.

Generally, in the process of the invention, the carboxy group of the compounds [IX(a), 1X(b)], XI and XIII, and the starting penem of process D will be protected in the respective process. Thus, for instance, the alkylation reaction of process A will give a carboxy protected derivative of the respective compound of formula I which has subsequently to be deprotected. The hydroxy group of the hydroxyalkyl attached to the 3-position of the azetidinone ring of the said compounds may also, conveniently, be protected.

Suitable hydroxy protecting groups for use in the process of this invention are such groups conventionally used for such purpose in the β-lactam art and which are readily removable by procedures utilizing elemental zinc or any other conventional procedures. Preferred hydroxy protecting groups are trichloroethoxycarbonyl, dimethyltributylsilyl, trimethylsilyloxycarbonyl and trimethylsilyl:trimethylsilyl is a particularly preferred protecting group and is removable by treatment with a mild aqueous acid, such as aqueous acetic acid.

Suitably carboxy protecting groups are those conventionally used in the penem art and which can be removed under conventional conditions without reaction with other functional groups present on the penem molecule, for example allylic, p-nitrobenzyl and trichloroethyl. The preferred carboxy protecting group is allyl.

The removal of the protecting group from a protected carboxyl group can be carried out by conventional procedures selected according to the identity of the protecting group. For the removal of the preferred protecting group, allyl, this can in general be effected under catalytic conditions in the presence of a base, preferably by utilizing procedures described in our European Patent Application Publication No. 0013663. Thus an allyl group is preferably removed by utilizing a suitable aprotic solvent, such as tetrahydrofuran, diethyl ether or methylene chloride, with an alkali metal alkylcarboxylate, preferably potassium or sodium 2-ethylhexanoate (to give the alkali metal penem salt, preferably the sodium or potassium penem salt directly) or carboxylic acid, preferably 2-ethylhexanoic acid (to give the penem free-acid) and a mixture of a palladium compound and triphenyl phosphine as a catalyst.

If the product is a zwitterion deprotection of the allylic group requires only the catalyst and any mild neucleophile (e.g. $H_2O$ or alcohol).

Preparation of the foregoing salts and esters may be carried out according to conventional procedures for forming salts of beta-lactams such as penicillins, cephalosporins and penems. Salts can be formed upon deprotection of an allyl group as above, or for example, by treating the free acid with metal compounds such as alkali metal salts of suitable carboxylic acids, or with ammonia or a suitable organic amine, wherein preferably stoichiometric amounts or only a small-excess of the salt-forming agent is used. Acid addition salts of the compound can be obtained in the usual manner, for example by treating a compound of formula I with an acid or a suitable anion exchange reagent. Zwitterions may be formed by neutralizing salts such as acid addition salts to the isoelectric point. The esters are preparable in a manner analogous to the preparation of the corresponding esters of penicillins and cephalosporins.

Salts may be converted in the usual manner into the free carboxy compounds.

The compounds may be prepared as their racemic mixtures, *e.g.*, a 5R,6S,8R compound is produced with its enantiomer (mirror image), *i.e.*, a 5S,6R,8S compound, in equal amounts when the starting compound is a racemic mixture. The two enantiomers may, if desired, be separated by conventinal means, *i.e.*, by fractional crystallizations of optically active salt forms, *i.e.*, the salts derived from optically active amino compounds, *i.e.*, (−)-brucine, or (+)- and (−)-ephedrine.

Alternatively, the compounds may be produced in optically active form by utilizing optically active starting material in the reacton procedures.

The starting materials for the procedure described above are either known in the art or can be prepared by standard methods.

The compounds of formula X in which $R^1$ is lower-alkyl substitututed by groups such as cyano or halogen may be prepared from compounds of the formula XIII

$$L-O-(\overset{\displaystyle R''}{\overset{|}{C}H})_n-Q \qquad XIII$$

in which R'' is hydroxyloweralkyl, n and Q are as defined above and L is a hydroxy protecting group. The hydroxy of the R'' group is mesylated e.g. by reaction with mesylchloride in dry pyridine at room temperature, and the mesyl group then displaced by reaction with an appropriate cyano or halogen containing reagent e.g. sodium cyanide or hydrogen fluoride. The protected oxygen can then be deprotected and displaced by or functionalized to the appropriate Z group e.g. it may be mesylated by reaction with mesylchloride as mentioned above for the R'' group, or converted to halide by reactio with a hydrogen halide.

The tautomeric compound of formula [IX(a),IX(b)] can be prepared by the methods disclosed in European Patent Application No. 83111615.7.

A description of a suitable procedure is given below in which for convenience compounds of 5R,6S,8R stereochemistry and a 6-(1-hydroxyethyl substituent is produced. It will be apparent to the skilled man, however, that compounds of other stereoconfigurations and other hydroxy-alkylsubstituents at the 6-position, as well as with carboxy protecting groups other than allyl, can be prepared using the same method.

The procedure, in a preferred form, comprises:

a) reacting an azetidinome of formula XX

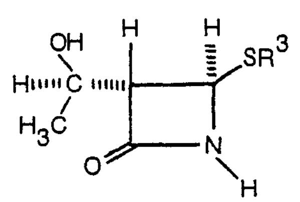

XX

in which $R^3$ is a sulfur protecting group, e.g. triphenylmethyl, with an α-substituted alkylacetate of formula XXI

$$WCH_2\overset{\displaystyle O}{\overset{\|}{C}}-OCH_2CH=CH_2 \qquad XXI$$

in which W is a leaving group e.g. iodo or bromo to form a compound of formula XXII

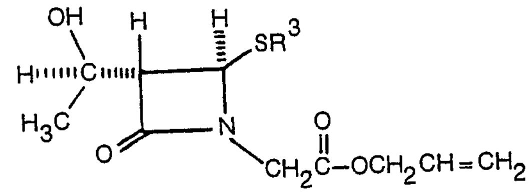

XXII

This reaction can be carried out at 15° to 30°C in the presence of an acid acceptor. Preferably the reaction is conducted in acetonitrile employing cesium carbonate or tetraalkyl ammonium hydroxide as the acid aceptor;

b) treating the compound of formula XXII with a stoichliometric excess of elemental zinc in hydrochloric acid in an suitable organic solvent such as tetrahydrofuran at 15°C to 25°, to form the compound of formula XXIII.

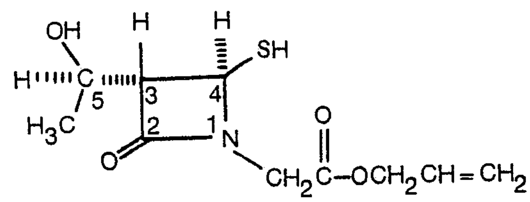

XXIII

c) protecting the hydroxy group at the 5 position with a trimethylsilyl group by reaction of compound of formula XIIII with bis-trimethyl silylacetamide; the reaction suitably being carried out in a solvent such as dimethylformamide at 0°C to 30°;

d) reacting the resulting protected compound with a thiocarbonyl compound of formula XXIV

$$S{=}C(Y)_2 \qquad XXIV$$

in which Y is a leaving group such as halogen naphthyloxy or imidazolyl, generally using the same solvent as in the preceding step as a temperature in the range 10°C to 45°C to form the compound XXV

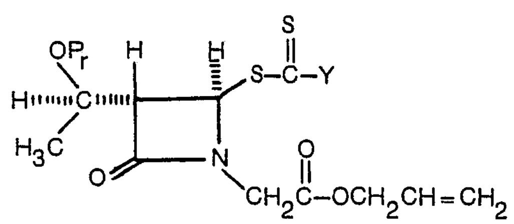

XXV

in which $P_r$ is trimethylsilyl and Y is as defined above;

c) treating compound XXV with an equimolar amount of a strong base such as lithium diisopropyl amide, to produce a compound [IX(a), IX(b)]; the rection will generally be carried out in an anhydrous inert organic solvent, such as tetrahydrofuran, with the base being added to a solution of compound XXV in the solvent; typical reaction temperatures are −50°C to −100°C, and the reaction will generally be complete within 5 minutes to 24 hours.

The compounds of formula XIII can be obtained by following the procedure described in European Patent Application publication No. 58317, e.g. by reacting of a compound of the formula

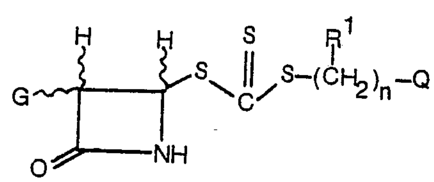

in which G, *n* and Q are as defined above, with a reactive derivative, e.g. chloride, of an acid of the formula

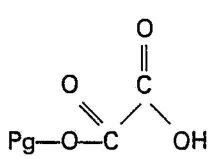

in which Pg is a carboxy protecting group as defined above. This reaction is usually carried out under normal acylating conditions, namely in an inert solvent and in the presence of an organic base, preferably a tertiary amine.

Some possible identities of Q are:

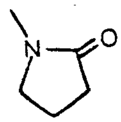
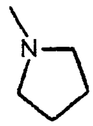
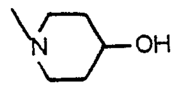
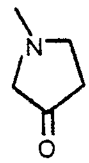
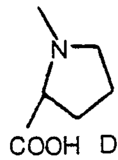
D+L
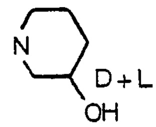
D+L
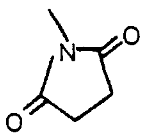
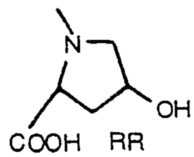
RR
RS
SS
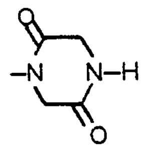
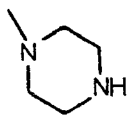
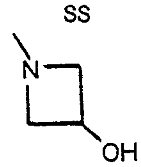
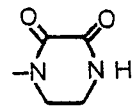
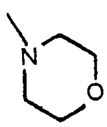
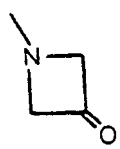
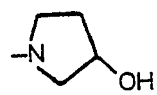
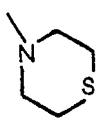
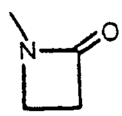
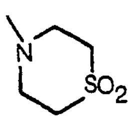
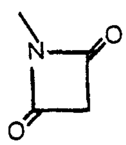
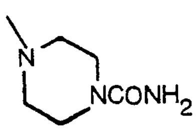
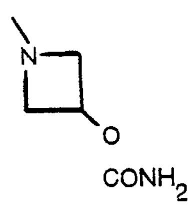

Typical examples of compounds of the present invention include compounds having the formula

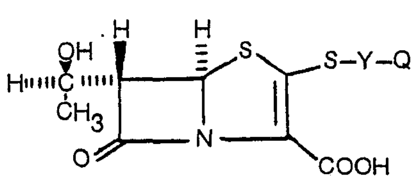

where —Y—Q is

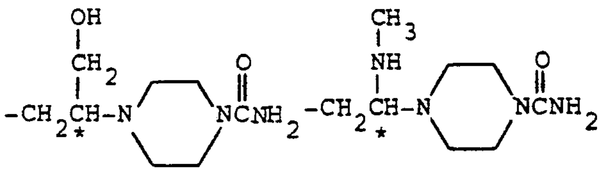

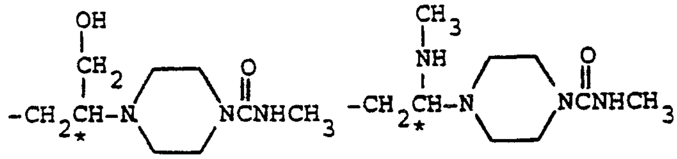

(* indicates a chiral centre of R,S or R, or S configuration) as well as the sodium or potassium salts or metabolisable esters of the foregoing.

Example 1

5R,6S,8R,2(RS)-6-(1-hydroxyethyl-2-[1-hydroxymethyl-2-(4-N-methylcarbamoyl-piperazin-1-yl)ethyl thio] penem-3 carboxylic acid

A) Dissolve 0.75 g of 1-ethoxy-1-(2-methylsulfonyloxy-3-(4-N-methylcarbamoyl-piperazin-1-yl)-propoxy)ethane in 30 ml methylene chloride and 0.83 ml dissopropylethylamine in 10 ml methylene chloride at 0°C.

Then add the resultant mixture to a solution of 1 g of allyl (5R,6S,8R)-2-thiol-6-(1-hydroxyethyl)penem-3-carboxylic acid and allyl (5R,6S,8R)-2-thione-6-(1-hydroxyethyl)penem-3-carboxylate in 10 ml tetrahydrofuran, 10 ml water, and 0.3 g sodium bicarbonate. Stir at room temperature until thin layer chromography (methanol:methylene chloride, 1:9) indicates no starting material is left. Evaporate the solvent *in vacuo* and partition the resultant residue between water and methylene chloride. Dry the organic layer over magnesium sulfate and evaporate the solvent *in vacuo*. Purify the resultant residue using silica gel, eluting with 25% ethyl acetate/methylene chloride → 100% ethyl acetate to obtain allyl (5R,6S,8R,2(RS) - 6 - (1 - hydroxyethyl - 2 - [1 - ethoxy - 1 - ethoxy) - methyl - 2 - (4 - N - methylcarbamoyl - piperazin - 1 - yl)ethyl)thio - penem - 3 - carboxylate.

B) Dissolve 174 mg of the product of step A in 10 ml of a mixture of water:tetrahydrofuran (1:1), then adjust to pH 3 with a 5% aqueous solution of p-toluenesulfonic acid. Let the mixture stand for 1.5 hours, evaporate the solvent *in vacuo* and partition the resultant residue between 5% aqueous sodium bicarbonate and methylene chloride. Evaporate the organic layer to obtain allyl (5R,6S,8R,2(RS) - 6 - (1 - hydroxyethyl - 2 - [1 - hydroxymethyl - 2 - (4 - N - methylcarbamoyl - piperazin - 1 - yl)ethyl]thio - penem - 3 - carboxylate

C) Under argon, redissolve the allyl ester product of step B in 10 ml methylene chloride; add 25 mg triphenyl phosphine, 45 mg 2-ethyl hexanoic acid, and 10 mg Pd° reagent. Allow the mixture to stand until the reaction is complete as shown by thin layer chromatography (5% methanol in methylene chloride as solvent). Extract the resultant product with 2 × 10 ml water, then wash the aqueous layer with 3 × 10 ml methylene chloride. Lyophilize the aqueous layer to obtain the crude title compound.

Purify the product by reverse phase column chromatography (25 g silica gel eluted with water) to obtain the title compound.

Example 2

(5R,6S,8R,2(RS)-6-(1-hydroxyethyl-2-[1-hydroxymethyl-2-(4-N-methylcarbamoyl-piperazin-1-yl)ethylthio] penem-3 carboxylic acid.

A) Dissolve 2 g of allyl (5R,6S,8R) - 2 - ethylthio - 6 - (1 - hydroxyethyl)penem - 3 - carboxylate in 80 ml methylenechloride at 5°C, add 2 g calcium carbonate, then l.1 g m-perbenzoic acid in 20 ml methylene chloride. After 20 minutes extract the reaction mixture with 100 ml water, separate and evaporate the organic layer to obtain allyl(5R,6S,8R) - 2 - ethylsulfinyl - 6 - (1 - hydroxyethyl)penem - 3 - carboxylate.

B) Dissolve 500 mg of the product of step A in a mixture of 4 ml tetrahydrofuran and 2 ml water, add 100 mg sodium bicarbonate, then 700 mg of the product of 1-ethoxy-1-(2-mercapto-3-(4-N-methylcarbamoyl-piperazin-yl)-propoxyethane and let stand 1 hour. Partition the resultant mixture between methylene chloride and water, evaporate the methylene chloride and purify the resultant residue on a silica gel column using methylene chloride-tetrahydrofuran (1:1) to obtain allyl (5R,6S,8R,2(R,S) - 2 - [1 - (ethoxy - 1 - ethoxy) - methyl - 2 - (4 - N - methylcarbamoyl - piperazin - 1 - yl) - ethylthio - 6 - (1 - hydroxyethyl)penem - 3 - carboxylate.

C) Treat the resulting allylester by the procedures described in Example 1 steps B and C to obtain the title compound.

By following the previously described procedures, using appropriate starting materials there is obtained compounds of the formula:

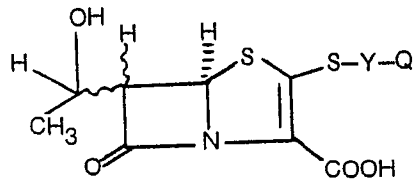

wherein Q is as defined above and Y (read from right to left) is

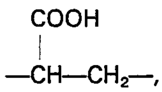, 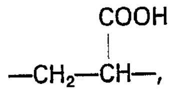, 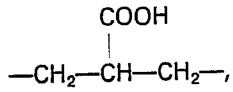,

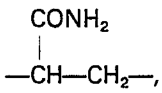, 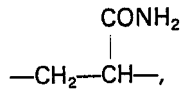, 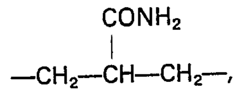,

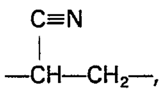, 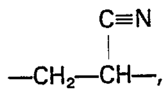, 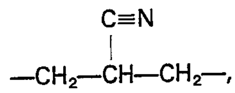,

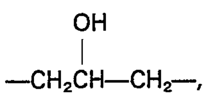, 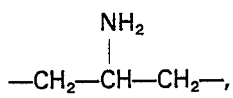, 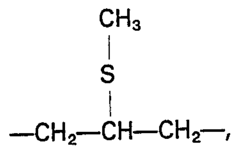,

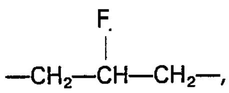, 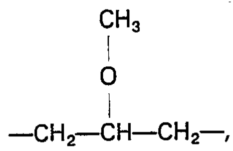, 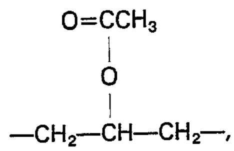,

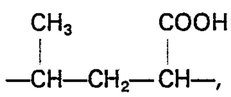, 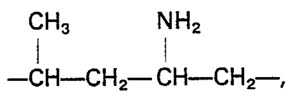, 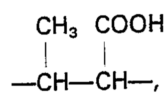,

In the following examples, the Active Ingredient is 5R,6S,8R, 2(RS) - 6 - (1 - hydroxyethyl) - 2 - [2' - hydroxymethyl - 2'(4 - N - methylcarbamoyl - piperazin - 1 - yl) - ethyl]thio - penem - 3 - carboxylic acid or an equivalent amount of any of its pharmaceutically acceptable salts and esters, or an equivalent amount of any other of the compounds of formula I as defined above.

Formulation 1

Injectable Solution

| Ingredients | mg/ml | mg/ml |
|---|---|---|
| Active Ingredient | 100 | 500 |
| Methylparaben | 1.8 | 1.8 |
| Propylparaben | 0.2 | 0.2 |
| Sodium Bisulfite | 3.2 | 3.2 |
| Disodium Edetate | 0.1 | 0.1 |
| Sodium Sulfate | 2.6 | 2.6 |
| Water for Injection q.s. ad | 1.0 ml | 1.0 ml |

Method of Manufacture

1. Dissolve parabens in a portion (85% of the final volume) of the water for injection at 65—70°C.
2. Cool to 25.35°C. Charge and dissolve the sodium bisulfite, disodium edetate and sodium sulfate.
3. Charge and dissolve the active ingredient.
4. Bring the solution to final volume by adding water for injection.
5. Filter the solution through 0.22μ membrane and fill into appropriate containers.
6. Terminally sterilize the units by autoclaving.

Formulation 2

Injectable Powder: (per vial)

| | c/vial | c/vial |
|---|---|---|
| Active ingredient | 0.5 | 1.0 |

Add sterile water for injection or bacteriostatic water for injection for reconstruction.

**Claims for the Contracting States: BE CH DE FR GB IT LI LU NL SE**

1. A compound having the formula

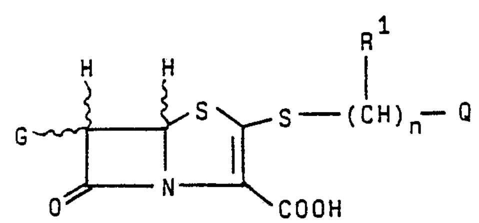

I

wherein
G is hydroxy, $C_1$—$C_6$ alkyl,
Q is the group

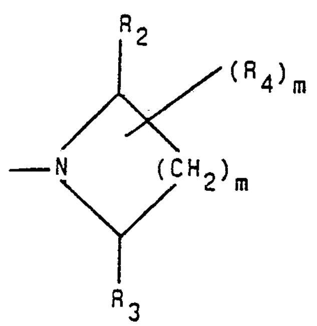

or

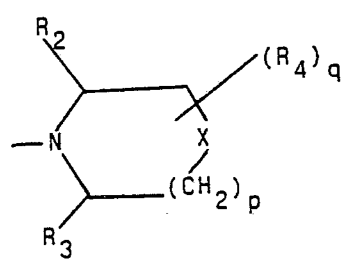

A B

wherein:
each $R^1$ is independently $C_1$—$C_6$ alkyl, hydrogen, carboxy, carbamyl, cyano, hydroxy, amino, $C_1$—$C_6$ alkylthio, fluoro, $C_1$—$C_6$ alkoxy, $C_1$—$C_6$ alkanoyloxy, or $C_1$—$C_6$ alkyl substituted by imidazolyl (which may be substituted by a group R defined below) amino, mono- or di-$C_1$—$C_6$ alkylamino, ureido, semicarbazido, hydroxy, cyano, halogen, $C_1$—$C_6$ alkoxy, carbamyloxy, carboxy, carbamyl, $C_1$—$C_6$ alkylcarbonyl, sulfo, sulfamyl, $C_1$—$C_6$ alkylsulfonyl, $C_1$—$C_6$ alkoxysulfonyl, $C_1$—$C_6$ alkylcarbonyl, hydroxy $C_1$—$C_6$ alkylcarbonyl or hydroxy $C_1$—$C_6$ alkylsulfonyl, with the proviso that when $R_1$ is attached to a carbon atom adjacent either to the sulphur atom connected to the 2-position of the penem ring or the nitrogen atom by which the group Q is connected to the side chain

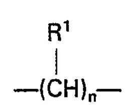

then such $R_1$ cannot be hydroxy, amino or fluoro;

R is hydrogen, $C_1$—$C_6$ alkyl, amino $C_1$—$C_6$ alkyl, mono- and di-$C_1$—$C_6$ alkylamino $C_1$—$C_6$ alkyl, carboxy $C_1$—$C_6$ alkyl, sulfo $C_1$—$C_6$ alkyl, hydroxy $C_1$—$C_6$ alkyl, cyano, hydroxy, amino, mono- and di-$C_1$—$C_6$

alkylamino, $C_1$—$C_6$ alkylsulfonate, sulfamyl, halogeno, hydroxylimino $C_1$—$C_6$ alkyl, $C_1$—$C_6$ alkoxyimino $C_1$—$C_6$ alkyl, carboxy, carbamyl, mono- or di-$C_1$—$C_6$ alkylcarbamyl, nitro, carbamyloxy, ureido, ureido $C_1$—$C_6$ alkyl, or carbamylhydrazo $C_1$—$C_6$ alkyl;

$R_2$ and $R_3$ are independently selected from hydrogen, $C_1$—$C_6$ alkyl, amino $C_1$—$C_6$ alkyl, mono- and di-$C_1$—$C_6$ alkylamino- $C_1$—$C_6$ alkyl, carboxy $C_1$—$C_6$ alkyl, carboxy, hydroxy $C_1$—$C_6$ alkyl, cyano, oxo, carbamyl, and mono- and di-$C_1$—$C_6$ alkyl- carbamyl;

$R_4$ is chosen from the groups listed for $R_2$ and $R_3$, sulfo-$C_1$—$C_6$ alkyl, hydroxy, amino, mono- and di-$C_1$—$C_6$ alkylamino, $C_1$—$C_6$ alkylsulfonate, sulfamyl, halogeno, hydroxylimino-$C_1$—$C_6$ alkyl, $C_1$—$C_6$ alkoxyimino $C_1$—$C_6$ alkyl, hydroxylimino and $C_1$—$C_6$ alkoxyimino,

X is O, S, $SO_2$, $NR_2$ or $NCOR_2$, wherein $R_2$ is as defined above,

m and n are 1 to 4

P is 1 or 2

q is 1, 2 or 3

and the pharmaceutically acceptable salts and pharmaceutically acceptable esters thereof.

2. A compound, as defined in claim 1, characterized in that G is 1-hydroxyethyl and the stereoconfiguration is 5R, 6S, 8R.

3. A compound as defined in claim 1 or 2, characterized in that $R^1$ is a substituted $C_1$—$C_6$ alkyl group as set forth in claim 1.

4. A compound as defined in claim 3, wherein $R^1$ is hydroxymethyl or aminomethyl.

5. A compound as defined in any one of claims 1 to 4 wherein Q is the grouping B, p is 1, $R_2$, $R_3$ and $R_4$ are hydrogen, and X is

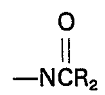

wherein $R_2$ is amino or $C_1$—$C_6$ alkylamino.

6. A pharmaceutical composition comprising a compound as defined in any one of the preceding claims, together with a pharmaceutically acceptable carrier or excipient.

**Claims for the Contracting State: AT**

1. A process for preparing a compound having the formula

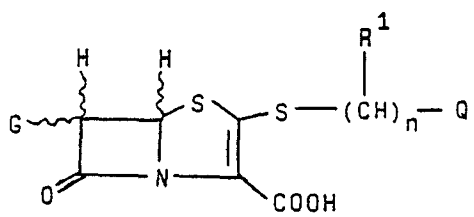

I

wherein

G is hydroxy $C_1$—$C_6$ alkyl,

Q is the group

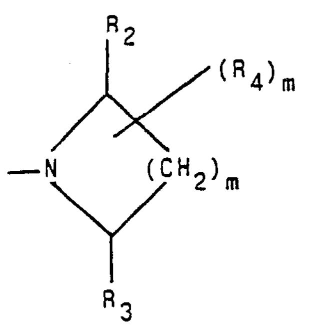

A

or

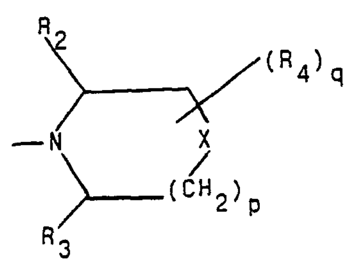

B

wherein:

each $R^1$ is independently $C_1$—$C_6$ alkyl, hydrogen, carboxy, carbamyl, cyano, hydroxy, amino, $C_1$—$C_6$ alkylthio, fluoro, $C_1$—$C_6$ alkoxy, $C_1$—$C_6$ alkanoyloxy, or $C_1$—$C_6$ alkyl substituted by imidazolyl (which may be

substituted by a group R defined below) amino, mono- or di-$C_1$—$C_6$ alkylamino, ureido, semicarbazido, hydroxy, cyano, halogen, $C_1$—$C_6$ alkoxy, carbamyloxy, carboxy, carbamyl, $C_1$—$C_6$ alkylcarbonyl, sulfo, sulfamyl, $C_1$—$C_6$ alkylsulfonyl, $C_1$—$C_6$ alkoxysulfonyl, $C_1$—$C_6$ alkylcarbonyl, hydroxy $C_1$—$C_6$ alkylcarbonyl or hydroxy $C_1$—$C_6$ alkylsulfonyl, with the proviso that when $R_1$ is attached to a carbon atom adjacent either to the sulphur atom connected to the 2-position of the penem ring or the nitrogen atom by which the group Q is connected to the side chain

$$-(\overset{R^1}{\overset{|}{CH}})_n-$$

then such $R_1$ cannot be hydroxy, amino or fluoro;

R is hydrogen, $C_1$—$C_6$ alkyl, amino $C_1$—$C_6$ alkyl, mono- and di-$C_1$—$C_6$ alkylamino $C_1$—$C_6$ alkyl, carboxy $C_1$—$C_6$ alkyl, sulfo $C_1$—$C_6$ alkyl, hydroxy $C_1$—$C_6$ alkyl, cyano, hydroxy, amino, mono- and di-$C_1$—$C_6$ alkylamino, $C_1$—$C_6$ alkylsulfonate, sulfamyl, halogeno, hydroxylimino $C_1$—$C_6$ alkyl, $C_1$—$C_6$ alkoxyimino $C_1$—$C_6$ alkyl, carboxy, carbamyl, mono- or di-$C_1$—$C_6$ alkylcarbamyl, nitro, carbamyloxy, ureido, ureido $C_1$—$C_6$ alkyl, or carbamylhydrazo $C_1$—$C_6$ alkyl;

$R_2$ and $R_3$ are independently selected from hydrogen, $C_1$—$C_6$ alkyl, amino $C_1$—$C_6$ alkyl, mono- and di-$C_1$—$C_6$ alkylamino- $C_1$—$C_6$ alkyl, carboxy $C_1$—$C_6$ alkyl, carboxy, hydroxy $C_1$—$C_6$ alkyl, cyano, oxo, carbamyl, and mono- and di-$C_1$—$C_6$ alkyl- carbamyl;

$R_4$ is chosen from the groups listed for $R_2$ and $R_3$, sulfo-$C_1$—$C_6$ alkyl, hydroxy, amino, mono- and di-$C_1$—$C_6$ alkylamino, $C_1$—$C_6$ alkylsulfonate, sulfamyl, halogeno, hydroxylimino-$C_1$—$C_6$ alkyl, $C_1$—$C_6$ alkoxyimino $C_1$—$C_6$ alkyl, hydroxylimino and $C_1$—$C_6$ alkoxyimino,

X is O, S, $SO_2$, $NR_2$ or $NCOR_2$, wherein $R_2$ is as defined above,

m and n are 1 to 4

P is 1 or 2

q is 1, 2 or 3 characterized in that

A) a compound of formula [IX(a), IX(b)]

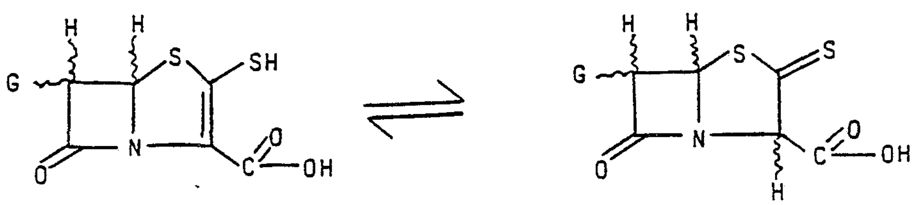

IX(a) IX(b)

in which G is as defined above, is transformed into a compound of formula I by introducing the group

$$-(\overset{R^1}{\overset{|}{CH}})_n-Q$$

(in which Q, *n* and $R^1$ are as previously defined) by

(i) Reacting a compound of formula X

$$Z^1-(\overset{R^1}{\overset{|}{CH}})_n-Q \qquad X$$

in which Q, $R^1$, and n are as defined above and $Z^1$ is a leaving group with the tautomer of formuale IX(a) and IX(b), or (ii) reacting a compound of formula $X^1$

$$\overbrace{CH_2 - CH}^{O}-(\overset{R^1}{\overset{|}{CH_2}})_{n^1}-Q \qquad X^1$$

in which Q and $R^1$ are as defined above and $n^1$ is 0 to 2 with the tautomer of formulae IX(a) and IX(b),

B) Reaction of a compound of the general formula XI

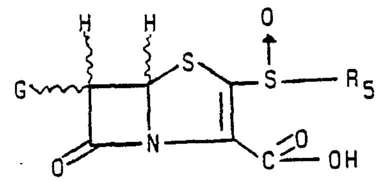

XI

in which G is as defined above and $R_5$ is an organic group, with a compound of general formula XII

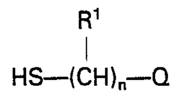

XII

in which Q, *n* and $R^1$ are as previously defined, or with a reactive derivative thereof, wherein the group $R_5$ is different from the group

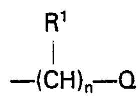

XII

by which it is replaced,

C) intramolecular cyclisation of a compound having the general formula XIII

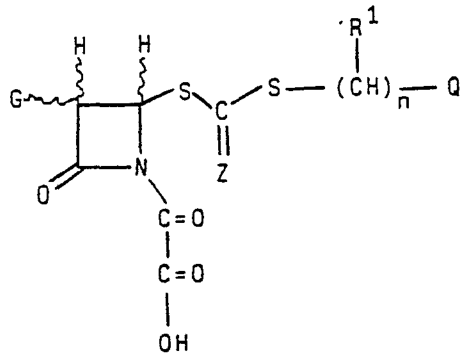

XIII

in which G, n, $R^1$ and Q are as defined above and Z is oxygen or sulphur, in the presence of a trivalent organophosphorous compound;

D) for the preparation of a compound of formula I in which at least one $R^1$ is cyano or fluoro, converting at least one hydroxyl group representing $R^1$ in a compound of formula I into the cyano or fluoro group; wherein the processes A, B, C or D, any functional groups are protected if necessary or desired, the process A, B, C, or D, being followed by removal of any protecting groups, before or after any desired separation of a stereoisomer, and isolation of the resulting penem compound of formula I as the free acid, pharmaceutically acceptable salt or pharmaceutically acceptable ester.

2. A process as defined in claim 1, characterized in that a compound of formula I in which G is 1-hydroxyethyl and the stereoconfiguration is 5R,6S,8R, is produced.

3. A process as defined in claim 1 or 2, characterized in that a compound of formula I in which $R^1$ is a substituted $C_1$—$C_6$ alkyl group as set forth in claim 1, is produced.

4. A process as defined in claim 3, wherein a compound of formula I in which $R^1$ is hydroxymethyl or aminomethyl, is produced.

5. A process as defined in any one of claims 1 to 4 in which a compound of formula I wherein Q is the grouping B, p is 1, $R_2$, $R_3$ and $R_4$ are hydrogen, and X is

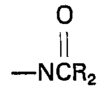

wherein $R_2$ is amino or $C_1$—$C_6$ alkylamino, is produced.

6. A process for the preparation of a pharmaceutical composition characterized in that a compound of formula I or a pharmaceutically acceptable salt or ester thereof, as defined in any of the preceding claims, is

mixed with a pharmaceutically acceptable carrier or excipient.

7. A process for the preparation of a pharmaceutical composition, characterized in that a compound of formula I or a pharmaceutically acceptable salt or ester thereof prepared by the process of any one of claims 1 to 5, is mixed with a pharmaceutically acceptable carrier or excipient.

**Patentansprüche für die Vertragsstaaten: BE CH DE FR GB IT LI LU NL SE**

1. Verbindung der Formel

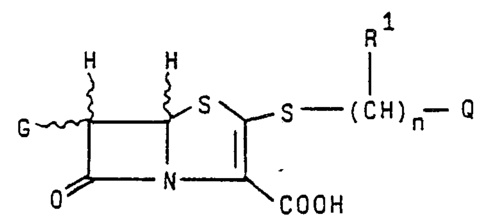

I

in der G Hydroxy-$C_1$—$C_6$-alkyl, und Q die Gruppe

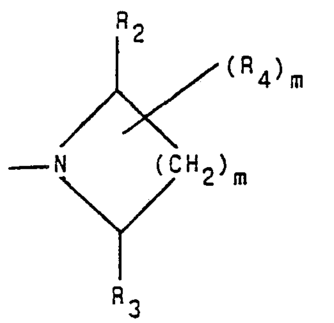

oder

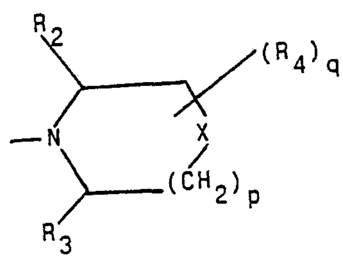

A B

ist,
worin jedes $R^1$, unabhängig voneinander, $C_1$—$C_6$-Alkyl, Wasserstoff, Carboxy, Carbamyl, Cyano, Hydroxy, Amino, $C_1$—$C_6$-Alkylthio, Fluor, $C_1$—$C_6$-Alkoxy, $C_1$—$C_6$-Alkanoyloxy, oder $C_1$—$C_6$-Alkyl, substituiert durch Imidazolyl (welches durch eine Gruppe R, wie sie unten definiert ist, substituiert sein kann) Amino, Mono- oder Di-$C_1$—$C_6$-alkylamino, Ureido, Semicarbazido, Hydroxy, Cyano, Halogen, $C_1$—$C_6$-Alkoxy, Carbamyloxy, Carboxy, Carbamyl, $C_1$—$C_6$-Alkylcarbonyl, Sulfo, Sulfamyl, $C_1$—$C_6$-Alkylsulfonyl, $C_1$—$C_6$-Alkoxysulfonyl, $C_1$—$C_6$-Alkoxycarbonyl, Hydroxy-$C_1$—$C_6$-alkylcarbonyl oder Hydroxy-$C_1$—$C_6$-alkylsulfonyl, ist, mit der Maßgabe, daß, wenn $R_1$ an ein Kohlenstoffatom gebunden ist, das entweder dem in 2-Stellung des Penemringes befindlichen Schwefelatom oder dem Stickstoffatom, über welches die Gruppe Q mit der Seitenkette

$$-\overset{\displaystyle R^1 \atop |}{(CH)_n}-$$

verknüpft ist, benachbart ist, $R_1$ nicht Hydroxy, Amino oder Fluor sein kann; R ist Wasserstoff, $C_1$—$C_6$-Alkyl, Amino-$C_1$—$C_6$-alkyl, Mono- und Di-$C_1$—$C_6$-alkylamino-$C_1$—$C_6$-alkyl, Carboxy-$C_1$—$C_6$-alkyl, Sulfo-$C_1$—$C_6$-alkyl, Hydroxy-$C_1$—$C_6$-alkyl, Cyano, Hydroxy, Amino, Mono- und Di-$C_1$—$C_6$-alkylamino, $C_1$—$C_6$-Alkylsulfonat, Sulfamyl, Halogen, Hydroxylimino-$C_1$—$C_6$-alkyl, $C_1$—$C_6$-Alkoxyimino-$C_1$—$C_6$-alkyl, Carboxy, Carbamyl, Mono- oder Di-$C_1$—$C_6$-alkyl-carbamyl, Nitro, Carbamyloxy, Ureido, Ureido-$C_1$—$C_6$-alkyl, oder Carbamylhydrazo-$C_1$—$C_6$-alkyl; $R_2$ und $R_3$ sind unabhängig voneinander Wasserstoff, $C_1$—$C_6$-Alkyl, Amino-$C_1$—$C_6$-alkyl, Mono- und Di-$C_1$—$C_6$-alkylamino-$C_1$—$C_6$-alkyl, Carboxy-$C_1$—$C_6$-alkyl, Carboxy, Hydroxy-$C_1$—$C_6$-alkyl, Cyano, Oxo, Carbamyl und Mono- und Di-$C_1$—$C_6$-alkyl-carbamyl; $R_4$ ist aus den für $R_2$ und $R_3$ angeführten Gruppen, Sulfo-$C_1$—$C_6$-alkyl, Hydroxy, Amino, Mono- und Di-$C_1$—$C_6$-alkylamino, $C_1$—$C_6$-Alkylsulfonat, Sulfamyl, Halogen, Hydroxylimino-$C_1$—$C_6$-alkyl, $C_1$—$C_6$-Alkoxyimino-$C_1$—$C_6$-alkyl, Hydroxylimino- und $C_1$—$C_6$-Alkoxyimino, ausgewählt, und X ist O, S, $SO_2$, $NR_2$ oder $NCOR_2$, wobei $R_2$ wie oben definiert ist, m und n sind 1 bis 4, P ist 1 oder 2, q ist 1, 2 oder 3, und die pharmazeutisch annehmbaren Salze und pharmazeutisch annehmbaren Ester davon.

2. Verbindung nach Anspruch 1, dadurch gekennzeichnet, daß G 1-Hydroxyethyl und die Stereokonfiguration 5R,6S,8R ist.

3. Verbindung nach Anspruch 1 oder 2, dadurch gekennzeichnet, daß $R^1$ eine substituierte $C_1$—$C_6$-Alkylgruppe, wie in Anspruch 1 angegeben, ist.

4. Verbindung nach Anspruch 3, in welcher $R^1$ Hydroxymethyl oder Aminomethyl ist.

5. Verbindung nach einem der Ansprüche 1 bis 4, in welcher Q die Gruppe B ist, p für 1 steht, $R_2$, $R_3$ und $R_4$ Wasserstoff sind, und X

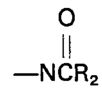

ist, worin $R_2$ Amino oder $C_1$—$C_6$-Alkylamino bedeutet.

6. Pharmazeutische Zusammensetzung, enthaltend eine Verbindung, wie sie in einem der vorhergehenden Ansprüche definiert ist, zusammen mit einem pharmazeutisch annehmbaren Träger oder Hilfsstoff.

**Patentansprüche für den Vertragsstaat: AT**

1. Verfahren zur Herstellung einer Verbindung der Formel

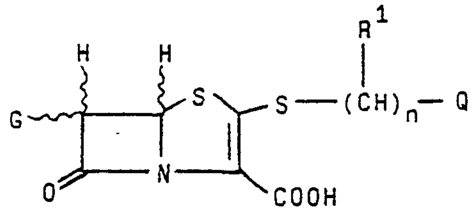

I

in der G Hydroxy-$C_1$—$C_6$-alkyl, und Q die Gruppe

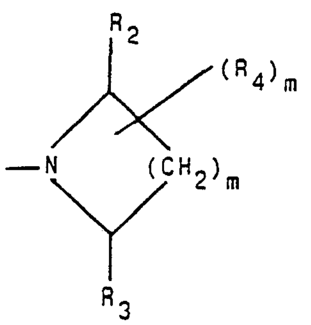

or

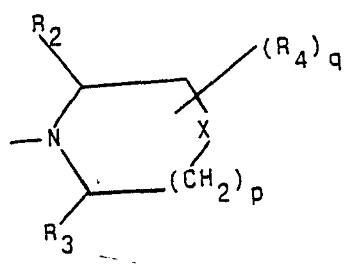

A B

ist,
worin jedes $R^1$, unabhängig voneinander, $C_1$—$C_6$-Alkyl, Wasserstoff, Carboxy, Carbamyl, Cyano, Hydroxy, Amino, $C_1$—$C_6$-Alkylthio, Fluor, $C_1$—$C_6$-Alkoxy, $C_1$—$C_6$-Alkanoyloxy, oder $C_1$—$C_6$-Alkyl, substituiert durch Imidazolyl (welches durch eine Gruppe R, wie sie unten definiert ist, substituiert sein kann) Amino, Mono- oder Di-$C_1$—$C_6$-alkylamino, Ureido, Semicarbazido, Hydroxy, Cyano, Halogen, $C_1$—$C_6$-Alkoxy, Carbamyloxy, Carboxy, Carbamyl, $C_1$—$C_6$-Alkylcarbonyl, Sulfo, Sulfamyl, $C_1$—$C_6$-Alkylsulfonyl, $C_1$—$C_6$-Alkoxysulfonyl, $C_1$—$C_6$-Alkoxycarbonyl, Hydroxy-$C_1$—$C_6$-alkylcarbonyl oder Hydroxy-$C_1$—$C_6$-alkylsulfonyl, ist, mit der Maßgabe, daß, wenn $R_1$ an ein Kohlenstoffatom gebunden ist, das entweder dem in 2-Stellung des Penemringes befindlichen Schwefelatom oder dem Stickstoffatom, über welches die Gruppe Q mit der Seitenkette

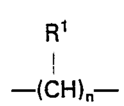

verknüpft ist, benachbart ist, R, nicht Hydroxy, Amino oder Fluor sein kann; R ist Wasserstoff, $C_1$—$C_6$-Alkyl, Amino-$C_1$—$C_6$-alkyl, Mono- und Di-$C_1$—$C_6$-alkylamino-$C_1$—$C_6$-alkyl, Carboxy-$C_1$—$C_6$-alkyl, Sulfo-$C_1$—$C_6$-alkyl, Hydroxy-$C_1$—$C_6$-alkyl, Cyano, Hydroxy, Amino, Mono- und Di-$C_1$—$C_6$-alkylamino, $C_1$—$C_6$-Alkylsulfonat, Sulfamyl, Halogen, Hydroxylimino-$C_1$—$C_6$-alkyl, $C_1$—$C_6$-Alkoxyimino-$C_1$—$C_6$-alkyl, Carboxy,

Carbamyl, Mono- oder Di-$C_1$—$C_6$-alkylcarbamyl, Nitro, Carbamyloxy, Ureido, Ureido-$C_1$—$C_6$-alkyl, oder Carbamylhydrazo-$C_1$—$C_6$-alkyl; $R_2$ und $R_3$ sind unabhängig voneinander Wasserstoff, $C_1$—$C_6$-Alkyl, Amino-$C_1$—$C_6$-alkyl, Mono- und Di-$C_1$—$C_6$-alkylamino-$C_1$—$C_6$-alkyl, Carboxy-$C_1$—$C_6$-alkyl, Carboxy, Hydroxy-$C_1$—$C_6$-alkyl, Cyano, Oxo, Carbamyl und Mono- und Di-$C_1$—$C_6$-alkyl-carbamyl; $R_4$ ist aus den für $R_2$ und $R_3$ angeführten Gruppen, Sulfo-$C_1$—$C_6$-alkyl, Hydroxy, Amino, Mono- und Di-$C_1$—$C_6$-alkylamino, $C_1$—$C_6$-Alkylsulfonat, Sulfamyl, Halogen, Hydroxylimino-$C_1$—$C_6$-alkyl, $C_1$—$C_6$-Alkoxyimino-$C_1$—$C_6$-alkyl, Hydroxylimino- und $C_1$—$C_6$-Alkoxyimino, ausgewählt, und X ist O, S, $SO_2$, $NR_2$ oder $NCOR_2$, wobei $R_2$ wie oben definiert ist, m und n sind 1 bis 4, P ist 1 oder 2, q ist 1, 2 oder 3, gekennzeichnet durch

A) Überführung einer Verbindung der Formel [IX(a), IX(b)]

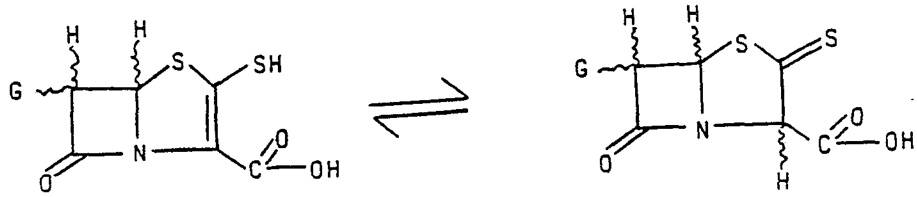

IX(a) IX(b)

in welcher G obige Bedeutung besitzt, in eine Verbindung der Formel I durch Einführung der Gruppe

$$-\overset{R^1}{\overset{|}{(CH)}}_n-Q$$

(in welcher Q, n und $R^1$ obige Bedeutung besitzen)

(i) durch Reaktion einer Verbindung der Formel

$$Z^1-\overset{R^1}{\overset{|}{(CH)}}_n-Q \qquad X,$$

in welcher Q, $R^1$ und n obige Bedeutung besitzen, und $Z^1$ eine Abgangsgruppe ist, mit der tautomeren Form der Formeln IX(a) und OX(b), oder

(ii) durch Reaktion einer Verbindung der Formel $X^1$

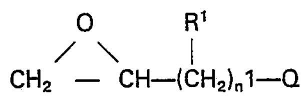

$X^1$,

in welcher Q und $R^1$ obige Bedeutung besitzen und $n^1$ für 0 bis 2 steht, mit der tautomeren Form der Formeln IX(a) und IX(b);

B) Reaktion einer Verbindung der allgemeinen Formel XI

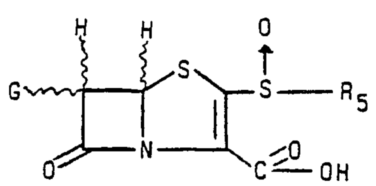

XI

in welcher G obige Bedeutung besitzt und $R_5$ eine organische Gruppe ist, mit einer Verbindung der allgemeinen Forem XII

$$HS-\overset{R^1}{\overset{|}{(CH)}}_n-Q \qquad XII,$$

in welcher Q, n und $R^1$ obige Bedeutung besitzen, oder mit einem reaktiven Derivat davon, wobei die Gruppe $R_5$ verschieden ist von der Gruppe

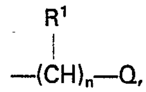

durch welche sie ersetzt wird;

C) intramolekulare Cyclisierung einer Verbindung mit der allgemeinen Formel XIII

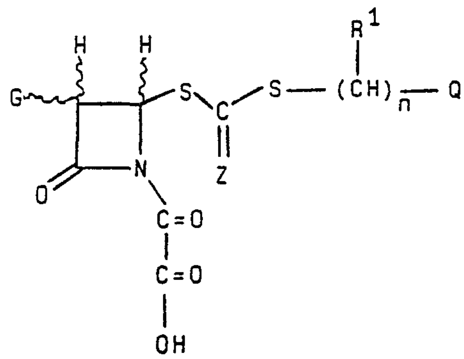

XIII,

in welcher G, n, $R^1$ und Q obige Bedeutung besitzen und Z für Sauerstoff oder Schwefel steht, in Gegenwart einer trivalenten Organophosphor-Verbindung;

D) zur Herstellung einer Verbindung der Formel I, in welcher mindestens ein $R^1$ Cyano oder Fluor ist, Umwandlung mindestens einer den Rest $R^1$ in einer Verbindnung der Formel I darstellenden Hydroxylgruppe in die Cyano- oder Fluorgruppe;

wobei in den Verfahren A, B, C oder D, funktionelle Gruppen, falls notwendig oder gewünscht, geschützt sind, und anschließend an das Verfahren A, B, C oder D die Entfernung der Schutzgruppen vor oder nach einer erwünschten Abtrennung eines Stereoisomeren erfolgt, und Isolierung der erhaltenen Penem-Verbindung der Formel I als freie Säure, pharmazeutisch annehmbares Salz oder pharmazeutisch annehmbarer Ester.

2. Verfahren nach Anspruch 1, dadurch gekennzeichnet, daß eine Verbindung der Formel I hergestellt wird, in der G 1-Hydroxyethyl und die Stereokonfiguration 5R,6S,8R ist.

3. Verfahren nach Anspruch 1 oder 2, dadurch gekennzeichnet, daß eine Verbindung der Formel I hergestellt wird, in der $R^1$ eine substituierte $C_1$—$C_6$-Alkylgruppe, wie in Anspruch 1 angegeben, ist.

4. Verfahren nach Anspruch 3, dadurch gekennzeichnet, daß eine Verbindung der Formel I hergestellt wird, in welcher $R^1$ Hydroxymethyl oder Aminomethyl ist.

5. Verfahren nach einem der Ansprüche 1 bis 4, dadurch gekennzeichnet, daß eine Verbindung der Formel I hergestellt wird, in welcher Q die Gruppe B ist, p für 1 steht, $R_2$, $R_3$ und $R_4$ Wasserstoff sind, und X

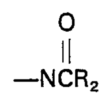

ist, worin $R_2$ Amino oder $C_1$—$C_6$-Alkylamino bedeutet.

6. Verfahren zur Herstellung einer pharmazeutischen Zusammensetzung, dadurch gekennzeichnet, daß eine Verbindung der Formel I oder ein pharmazeutisch annehmbares Salz oder Ester davon, wie in einem der vorstehenden Ansprüche definiert, mit einem pharmazeutisch annehmbaren Träger oder Hilfsstoff vermischt wird.

7. Verfahren zur Herstellung einer pharmazeutischen Zusammensetzung, dadurch gekennzeichnet, daß eine Verbindung der Formel I oder ein pharmazeutisch annehmbares Salz oder Ester davon, hergestellt durch ein Verfahren nach einem der Ansprüche 1 bis 5, mit einem pharmazeutisch annehmbaren Träger oder Hilfsstoff vermischt wird.

**Revendications pour les Etats contractants: BE CH DE FR GB IT LI LU NL SE**

1. Composé ayant la formule

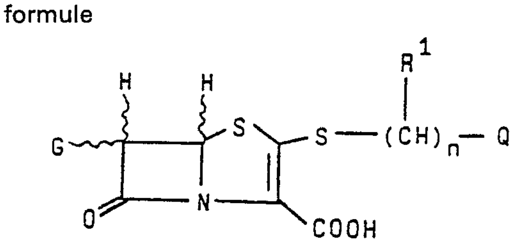

I

où

G est hydroxy-alcoyle $C_1$—$C_6$,
Q est le groupe

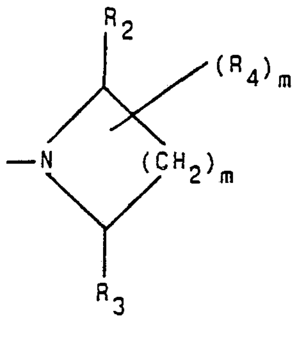

ou

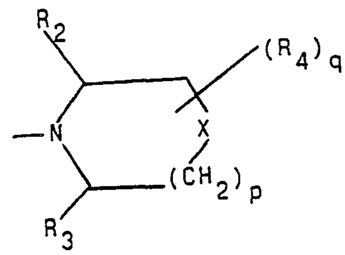

A B

où

chaque $R^1$ est indépendamment alcoyle $C_1$—$C_6$, hydrogène, carboxy, carbamyle, cyano, hydroxy, amino, alkylthio $C_1$—$C_6$, fluoro, alcoxy $C_1$—$C_6$, alcanoyloxy $C_1$—$C_6$ ou alcoyle $C_1$—$C_6$ substitué par imidazolyle (qui peut être substitué par un groupe R défini ci-dessous) amino, mono- ou di-alcoylamino $C_1$—$C_6$, uréido, semicarbazido, hydroxy, cyano, halogène, alcoxy $C_1$—$C_6$, carbamyloxy, carboxy, carbamyle, alcoxy $C_1$—$C_6$-carbonyle, sulfo, sulfamyle, alkyl $C_1$—$C_6$ sulfonyle, alcoxy $C_1$—$C_6$ sulfonyle, alcoxy $C_1$—$C_6$ carbonyle, hydroxy-alkyl $C_1$—$C_6$ carbonyle ou hydroxy-alkyl $C_1$—$C_6$ sulfonyle, à condition que lorsque $R_1$ est attaché à un atome de carbone adjacent soit à l'atome de soufre connecté à la position 2 du noyau pénèm ou à l'atome d'azote par lequel le groupe Q est connecté à la chaîne latérale

$$-(\overset{R^1}{\overset{|}{CH}})_n-$$

alors $R_1$ ne puisse être hydroxy, amino ou fluoro;

R est hydrogène, alcoyle $C_1$—$C_6$, amino-alcoyle $C_1$—$C_6$, mono- et di-alkylamino $C_1$—$C_6$ alcoyle $C_1$—$C_6$, carboxy-alcoyle $C_1$—$C_6$, sulfo alcoyle $C_1$—$C_6$, hydroxy-alcoyle $C_1$—$C_6$, cyano, hydroxy, amino, mono- et di-alkylamino $C_1$—$C_6$, alkyl $C_1$—$C_6$ sulfonate, sulfamyle, halogéno, hydroxylimino-alcoyle $C_1$—$C_6$, alcoxyimino $C_1$—$C_6$-alcoyle $C_1$—$C_6$, carboxy, carbamyle, mono- ou di-alkyl $C_1$—$C_6$ carbamyle, nitro, carbamyloxy, uréido, uréido-alcoyle $C_1$—$C_6$ ou carbamylhydrazo-alcoyle $C_1$—$C_6$;

$R_2$ et $R_3$ sont indépendamment choisis parmi hydrogène, alcoyle $C_1$—$C_6$, amino-alcoyle $C_1$—$C_6$, mono- et di-alcoylamino $C_1$—$C_6$-alcoyle $C_1$—$C_6$, carboxy-alcoyle $C_1$—$C_6$, carboxy, hydroxy-alcoyle $C_1$—$C_6$, cyano, oxo, carbamyle, et mono- et di-alkyl $C_1$—$C_6$ carbamyle;

$R_4$ est choisi dans les groupes dont la liste est donnée pour $R_2$ et $R_3$, sulfo-alcoyle $C_1$—$C_6$, hydroxy, amino, mono- et di-alkylamino $C_1$—$C_6$, alkylsulfonate $C_1$—$C_6$, sulfamyle, halogéno, hydroxylimino-alcoyle $C_1$—$C_6$, alcoxyimino $C_1$—$C_6$-alcoyle $C_1$—$C_6$, hydroxylimino et alcoxyimino $C_1$—$C_6$,

X est O, S, $SO_2$, $NR_2$ ou $NCOR_2$, où $R_2$ est tel que défini ci-dessus,

m et n sont 1 à 4,

P est 1 ou 2

q est 1, 2 ou 3.

et leurs sels acceptables en pharmacie et leurs esters acceptables en pharmacie.

2. Composé défini à la revendication 1, caractérisé en ce que G est 1-hydroxyéthyle et la stéréoconfiguration est 5R,6S,8R.

3. Composé selon la revendication 1 ou 2, caractérisé en ce que $R^1$ est un groupe alcoyle $C_1$—$C_6$ substitué tel qu'indiqué à la revendication 1.

4. Composé selon la revendication 3, où $R^1$ est hydroxyméthyle ou aminométhyle.

5. Composé selon l'une quelconque des revendications 1 à 4, où Q est le groupement B, p est 1, $R_2$, $R_3$ et $R_4$ sont hydrogène et X est

$$-N\overset{O}{\overset{\|}{C}}R_2$$

où $R_2$ est amino ou alcoylamino $C_1$—$C_6$.

6. Composition pharmaceutique comprenant un composé tel que défini selon l'une quelconque des revendications précédentes avec un véhicule ou excipient acceptable en pharmacie.

**Revendications pour l'Etat contractant: AT**

1. Procédé pour la préparation d'un composé ayant la formule

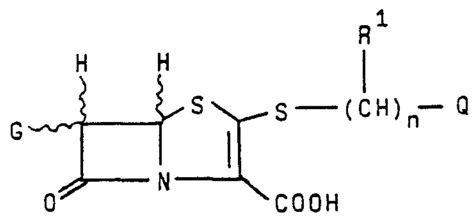

I

où

G est hydroxy-alcoyle $C_1$—$C_6$,

Q est le groupe

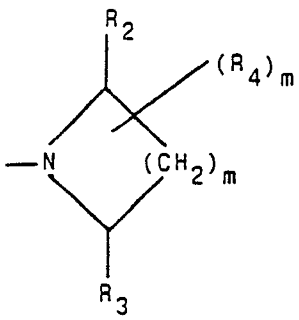

A

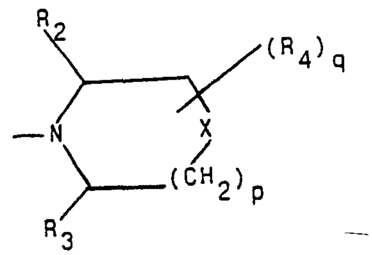

B

où

chaque $R^1$ est indépendamment alcoyle $C_1$—$C_6$, hydrogène, carboxy, carbamyle, cyano, hydroxy, amino, alkylthio $C_1$—$C_6$, fluoro, alcoxy $C_1$—$C_6$, alcanoyloxy $C_1$—$C_6$ ou alcoyle $C_1$—$C_6$ substitué par imidazolyle (qui peut être substitué par un groupe R défini ci-dessous) amino, mono- ou di-alcoylamino $C_1$—$C_6$, uréido, semicarbazido, hydroxy, cyano, halogène, alcoxy $C_1$—$C_6$, carbamyloxy, carboxy, carbamyle, alcoxy $C_1$—$C_6$-carbonyle, sulfo, sulfamyle, alkyl $C_1$—$C_6$ sulfonyle, alcoxy $C_1$—$C_6$ sulfonyle, alcoxy $C_1$—$C_6$ carbonyle, hydroxy-alkyl $C_1$—$C_6$ carbonyle ou hydroxy-alkyl $C_1$—$C_6$ sulfonyle, à condition que lorsque $R_1$ est attaché à un atome de carbone adjacent soit à l'atome de soufre connecté à la position 2 du noyau pénèm ou à l'atome d'azote par lequel le groupe Q est connecté à la chaîne latérale

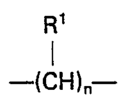

alors $R_1$ ne puisse être hydroxy, amino ou fluoro;

R est hydrogène, alcoyle $C_1$—$C_6$, amino-alcoyle $C_1$—$C_6$, mono- et di-alkylamino $C_1$—$C_6$ alcoyle $C_1$—$C_6$, carboxy-alcoyle $C_1$—$C_6$, sulfo alcoyle $C_1$—$C_6$, hydroxy-alcoyle $C_1$—$C_6$, cyano, hydroxy, amino, mono- et di-alkylamino $C_1$—$C_6$, alkyl $C_1$—$C_6$ sulfonate, sulfamyle, halogéno, hydroxylimino-alcoyle $C_1$—$C_6$, alcoxyimino $C_1$—$C_6$ alcoyle $C_1$—$C_6$, carboxy, carbamyle, mono- ou di-alkyl $C_1$—$C_6$ carbamyle, nitro, carbamyloxy, uréido, uréido-alcoyle $C_1$—$C_6$ ou carbamylhydrazo-alcoyle $C_1$—$C_6$;

$R_2$ et $R_3$ sont indépendamment choisis parmi hydrogène, alcoyle $C_1$—$C_6$, amino-alcoyle $C_1$—$C_6$, mono- et di-alcoylamino $C_1$—$C_6$-alcoyle $C_1$—$C_6$, carboxy-alcoyle $C_1$—$C_6$, carboxy, hydroxy-alcoyle $C_1$—$C_6$, cyano, oxo, carbamyle, et mono- et di-alkyl $C_1$—$C_6$ carbamyle;

$R_4$ est choisi dans les groupes dont la liste est donnée pour $R_2$ et $R_3$, sulfo-alcoyle $C_1$—$C_6$, hydroxy, amino, mono- et di-alkylamino $C_1$—$C_6$, alkylsulfonate $C_1$—$C_6$, sulfamyle, halogéno, hydroxylimino-alcoyle $C_1$—$C_6$, alcoxyimino $C_1$—$C_6$-alcoyle $C_1$—$C_6$, hydroxylimino et alcoxyimino $C_1$—$C_6$,

X est O, S, $SO_2$, $NR_2$ ou $NCOR_2$, où $R_2$ est tel que défini ci-dessus,

m et n sont 1 à 4,

P est 1 ou 2

q est 1, 2 ou 3, caractérisé en ce que

A) un composé de formule [IX(a), IX(b)]

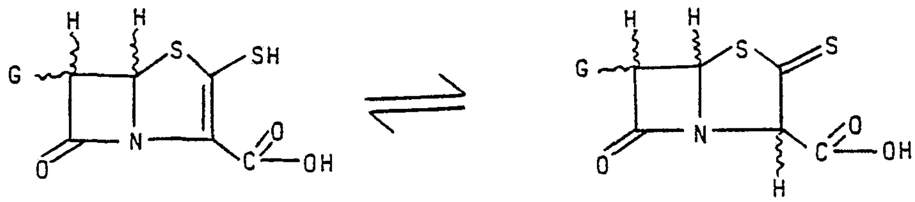

IX(a) IX(b)

où G est tel que défini ci-dessus, est transformé en un composé de formule I par introduction du groupe

$$-\overset{R^1}{\overset{|}{(CH)}}_n-Q$$

(où Q, n et $R^1$ sont tels que précédemment définis) par

(i) la réaction d'un composé de formule X

$$Z^1-\overset{R^1}{\overset{|}{(CH)}}_n-Q \qquad X$$

où Q, $R^1$ et n sont tels que définis ci-dessus et $Z^1$ est un groupe partant avec le tautomère des formules IX(a) et IX(b), ou

(ii) la réaction d'un composé de formule $X^1$

$$CH_2 \overset{O}{\diagup\diagdown} CH-\overset{R^1}{\overset{|}{(CH_2)}}_{n^1}-Q \qquad X^1$$

où Q et $R^1$ sont tels que définis ci-dessus et $n^1$ est 0 à 2 avec le tautomère des formules IX(a) et IX(b),

B) La réaction d'un composé de formule générale XI

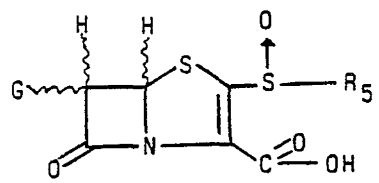

XI

où G est tel que défini ci-dessus et $R_5$ est un groupe organique, avec un composé de formule générale XII

$$HS-\overset{R^1}{\overset{|}{(CH)}}_n-Q \qquad XII$$

où Q, n et R' sont tels que précédemment définis ou avec son dérivé réactif, où le groupe $R_5$ est différent du groupe

$$-\overset{R^1}{\overset{|}{(CH)}}_n-Q$$

par lequel il est remplacé,

C) une cyclisation intramoléculaire d'un composé ayant la formule générale XIII

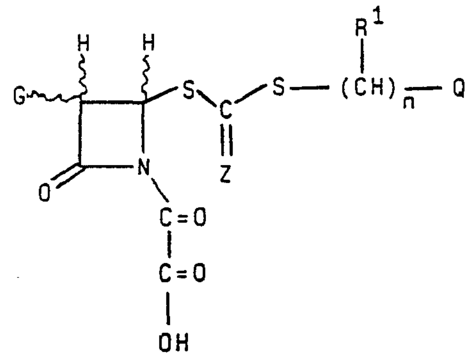

XIII

où G, n, $R^1$ et Q sont tels que définis ci-dessus et Z est oxygène ou soufre, en présence d'un composé organophosphoreux trivalent,

D) pour la préparation d'un composé de formule I où au moins un $R^1$ est cyano ou fluoro, la conversion d'au moins un groupe hydroxyle représentant $R^1$ dans un composé de formule I en le groupe cyano ou fluoro:

où dans les procédés A, B, C ou D, tous les groupes fonctionnels sont protégés si nécessaire ou souhaité; le procédé A, B, C ou D étant suivi par l'enlèvement de tout groupe protecteur, avant ou après toute séparation souhaitée d'un stéréoisomère et isolement du composé pénème résultant de formule I sous forme de l'acide libre, du sel acceptable en pharmacie ou de l'ester acceptable en pharmacie.

2. Procédé tel que défini à la revendication 1, caractérisé en ce qu'un composé de formule I où G est 1-hydroxyéthyle et la stéréoconfiguration est 5R,6S,8R est produit.

3. Procédé tel que défini à la revendication 1 ou 2, caractérisé en ce qu'un composé de formule I où $R^1$ est un groupe alcoyle $C_1$—$C_6$ substitué tel qu'indiqué à la revendication 1 est produit.

4. Procédé tel que défini à la revendication 3, où un composé de formule I où $R^1$ est hydroxyméthyle ou aminométhyle est produit.

5. Procédé tel que défini selon l'une quelconque des revendications 1 à 4, où un composé de formule I où Q est le groupement B, p est 1, $R_2$, $R_3$ et $R_4$ sont de l'hydrogène et X est

$$-N\overset{O}{\overset{\|}{C}}R_2$$

où $R_2$ est amino ou alkylamino $C_1$—$C_6$, est produit.

6. Procédé pour la préparation d'une composition pharmaceutique, caractérisé en ce qu'un composé de formule I ou son sel ou ester acceptable en pharmacie, selon l'une quelconque des revendications précédentes, est mélangé à un véhicule ou excipient acceptable en pharmacie.

7. Procécé pour la préparation d'une composition pharmaceutique, caractérisé en ce qu'un composé de formule I ou son sel ou ester acceptable en pharmacie préparé par le procédé selon l'une quelconque des revendication 1 à 5 est mélangé à un véhicule ou excipient acceptable en pharmacie.